# EUROPEAN PATENT APPLICATION

(11) **EP 4 023 217 A1**
(43) Date of publication of application: **06.07.2022**
(21) Application number: 21217827.1
(22) Date of filing: 27.12.2021
(51) Int. Cl.: A61K 9/48

(54) **PHARMACEUTICAL CAPSULE COMPOSITIONS OF ALOGLIPTINE**

(30) Priority: 31.12.2020 TR 202022612
(71) Applicant: Sanovel Ilac Sanayi ve Ticaret A.S., 34460 Istanbul (TR)
(72) Inventor: KOKSAL UZUN, Selin, Istanbul (TR); BASARAN, Salih Sermet, Istanbul (TR); DEMIR, Bülent, Istanbul (TR); TOK, Gülcin, Istanbul (TR); SÜNEL, Fatih, Istanbul (TR)
(74) Representative: Sevinç, Erkan

(57) **Abstract**

The present invention relates to pharmaceutical compositions comprising alogliptin or a pharmaceutically acceptable salt thereof and at least one pharmaceutically acceptable excipient.

## Description

### Field of the invention

The present invention relates to pharmaceutical compositions comprising alogliptin or a pharmaceutically acceptable salt thereof and a pharmaceutically acceptable excipient.

### Background of the invention

Diabetes mellitus is a group of disorders of carbohydrate metabolism in which the action of insulin is diminished or absent through altered secretion and/or the insulin activity is decreased. Two main types of diabetes are Type 1 and Type 2:
Type 1 diabetes occurs because the insulin-producing cells of the pancreas (beta cells) are damaged. With Type 1 diabetes, the pancreas makes little or no insulin, so sugar cannot get into the body's cells for use as energy. People with Type 1 diabetes must use insulin injections to control their blood glucose.

In Type 2 diabetes, the pancreas produces insulin, but the produced insulin is either insufficient or ineffective. This type of diabetes occurs mostly in people who are over the age of 40 years and overweight. Type 2 diabetes may sometimes be controlled with a combination of diet, weight management, and exercise. However, treatment also may include oral glucose-lowering medications or insulin injections.

Alogliptin, 2-[[6-[(3-aminopiperidin-1-yl)-3-methyl-2,4-dioxopyrimidin-1-yl]methyl]benzonitrile, is an orally administered antidiabetic drug, having the chemical structure shown by Formula I

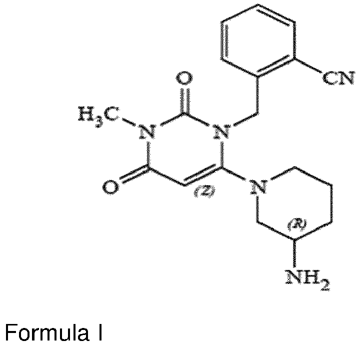

Alogliptin is disclosed in patent document no's. US 2005/261271, EP 1586571 and also WO 2005/095381. Patent document no. WO 2007/035629 discloses alogliptin in the form of the benzoate salt, hydrochloride salt, or tosylate salt. Polymorphs of alogliptin benzoate are disclosed in patent document no. WO 2007/035372. A process for preparing alogliptin is disclosed in patent document nos. WO 2007/112368 and WO 2007/035629.

Stability, solubility and dissolution rate problems are seen in formulations in the prior art. In addition, there are problems encountered during the compression process in the production of tablets. Currently there is a need for developing an improved oral pharmaceutical composition of alogliptin which has content uniformity, high solubility, and thus high bioavailability and long-term stability.

Therefore, there is an outstanding need for a new pharmaceutical composition preferably comprising alogliptin.

### Detailed Description of the Invention

The main object of the present invention is to provide pharmaceutical compositions with high solubility, high bioavailability, high physical and chemical stability and a long shelf life.

Another object of the present invention is to provide pharmaceutical compositions with high content uniformity.

The term "alogliptin" as used herein means alopgliptin in free base form or in the form of pharmaceutically acceptable salt, crystalline polymorph, solvate, hydrate or ester or in an amorphous form or in the form of mixtures thereof.

Alogliptin is poorly soluble in water (10 mg/ml at 25 °C) and exhibits relatively low oral bioavailability.

In the present invention, the pharmaceutical composition is suitable for oral administration. Solid oral dosage form is used for bioavailability. Suitable solid oral dosage forms are selected from the group consisting of tablets, capsules, granules, powders, pellets or unit dosage packs.

The pharmaceutical compositions of the present invention comprise alogliptin or pharmaceutically acceptable salts, polymorphs, solvates, hydrates or esters thereof and croscarmellose sodium.

According to one embodiment of the present invention, the composition further comprises at least one pharmaceutically acceptable excipient selected from among diluents, binders, disintegrants, lubricants, glidants or mixtures thereof.

According to one embodiment of the present invention, the ratio of the said binders to disigrants is between 0.2 and 1.3, preferably between 0.25 and 1.2, more preferably between 0.3 and 1.1.

According to one embodiment of the present invention, the said binders are hydroxy propyl cellulose and/or hydroxy propyl methyl cellulose and/or polyvinyl pyrrolidone. Mixtures of these in appropriate amounts may also be preferred.

According to one embodiment of the present invention, the said disintegrant is croscarmellose sodium and/or starch. Starch is pregelatinized starch and/or sodium starch glycolate. Mixtures of these in appropriate amounts may also be preferred.

According to one embodiment of the present invention, the said capsule is gelatin.

According to one embodiment of the present invention, the said capsule is hydroxy propyl methyl cellulose.

A capsule composition according to a preferred embodiment comprising comprises:
a. 1 - 15% by weight of alogliptin
b. 1 - 85% by weight of mannitol and/or microcrystalline cellulose
c. 1 - 15% by weight of croscarmellose sodium and/or starch
d. 1 - 70% by weight of hydroxypropyl cellulose and/or hydroxypropyl methyl cellulose and/or polyvinyl pyrrolidone
e. 0.5 - 5% by weight of colloidal silicon dioxide
f. 0.1 - 5% by weight of magnesium stearate

According to one embodiment of the present invention, diluent is selected from a group comprising microcrystalline cellulose, mannitol, lactose, starch, calcium phosphate dihydrate, calcium phosphate trihydrate, dibasic calcium phosphate, calcium carbonate, calcium sulfate, carboxymethyl cellulose calcium, powdered cellulose, cellulose acetate, pregelatinized starch, lactose monohydrate, corn starch, or mixtures thereof.

Compositions of the invention may be developed in an oral dosage form with immediate release, extended release, sustained release, controlled release, modified release and delayed release, or a combination thereof. Such compositions may be prepared using rate controlling polymers.

### Example 1

| **Ingredients** | **Excipient description** | **(%) Amount (w/w)** |
|---|---|---|
| Alogliptin benzoate | Active ingredient | 1- 15 |
| Mannitol | Filler | 20 - 70 |
| Microcrystalline cellulose | Filler | 10 - 20 |
| Hydroxypropyl cellulose | Binder | 1 -10 |
| Croscarmellose sodium | Disintegrant | 1 -10 |
| Colloidal silicon dioxide | Glidant | 0.5 - 5 |
| Magnesium stearate | Lubricant | 0.1 - 5 |
| **Total** | | **100** |
| Gelatin or Hydroxy propyl methyl cellulose Capsule | | |

### Example 2

| **Ingredients** | **Excipient description** | **(%) Amount (w/w)** |
|---|---|---|
| Alogliptin benzoate | Active ingredient | 1- 15 |
| Mannitol | Filler | 20 - 70 |
| Microcrystalline cellulose | Filler | 10 - 20 |
| Hydroxy propyl methyl cellulose and/or polyvinyl pyrrolidone | Binder | 1 - 10 |
| Croscarmellose sodium | Disintegrant | 1 - 10 |
| Colloidal silicon dioxide | Glidant | 0.5 - 5 |
| Magnesium stearate | Lubricant | 0.1 - 5 |
| **Total** | | **100** |
| Gelatin or Hydroxy propyl methyl cellulose Capsule | | |

### Example 3

| **Ingredients** | **Excipient description** | **(%) Amount (w/w)** |
|---|---|---|
| Alogliptin benzoate | Active ingredient | 1- 15 |
| Mannitol | Filler | 20 - 70 |
| Microcrystalline cellulose | Filler | 10 - 20 |
| Hydroxy propyl methyl cellulose | Binder | 1 - 10 |
| Croscarmellose sodium | Disintegrant | 1 - 10 |
| Colloidal silicon dioxide | Glidant | 0.5 - 5 |
| Magnesium stearate | Lubricant | 0.1 - 5 |
| **Total** | | **100** |
| Gelatin or Hydroxy propyl methyl cellulose Capsule | | |

The pharmaceutical formulations of the invention are prepared using the following steps in detail:
- Granulating the mixture consisting of all of alogliptin, mannitol, hydroxypropylcellulose and microcrystalline cellulose and a part of the croscarmellose sodium with water,
- drying and grinding the granules,
- adding microcrystalline cellulose, the remainder of croscarmellose sodium and colloidal silicon dioxide to the outer phase and mixing,
- adding magnesium stearate to the mixture and then obtaining the final mixture,
- performing the capsule filling process.

A composition with the advantage of content uniformity and stability is obtained due to the said gelatin or hydroxypropyl methyl cellulose capsules and binders, disintegrants. In addition, a composition advantageous in terms of dissolution rate and solubility is obtained due to the said binders and disintegrants.

The formulation is administered in a dosage form of tablets of 6.25 mg, 12.5 mg and 25 mg. Capsule sizes are selected from available capsules in the prior art according to the dosage form.

## Claims

1. The invention is a pharmaceutical capsule composition, comprising alogliptin or pharmaceutically acceptable salts, polymorphs, solvates, hydrates or esters thereof and croscarmellose sodium.

2. A pharmaceutical composition according to claim 1, further comprising at least one pharmaceutically acceptable excipient selected from among diluents, binders, disintegrants, lubricants, glidants or mixtures thereof.

3. A pharmaceutical composition according to claim 2, wherein the ratio of said binders to disintegrants is between 0.2 and 1.3, preferably between 0.25 and 1.2, more preferably between 0.3 and 1.1.

4. A pharmaceutical composition according to claim 2, wherein the said binders are hydroxy propyl cellulose and/or hydroxy propyl methyl cellulose and/or polyvinyl pyrrolidone.

5. A pharmaceutical composition according to claim 2, wherein the said disintegrant is croscarmellose sodium and/or starch.

6. A pharmaceutical composition according to claim 1, wherein the said capsule is gelatin.

7. A pharmaceutical composition according to claim 1, wherein the said capsule is hydroxy propyl methyl cellulose.

8. A pharmaceutical capsule composition according to any one of the preceding claims, comprising
a. 1-15% by weight of alogliptin
b. 1-85% by weight of mannitol and/or microcrystalline cellulose
c. 1-15% by weight of croscarmellose sodium and/or starch
d. 1-70% by weight of hydroxypropyl cellulose and/or hydroxypropyl methyl cellulose and/or polyvinyl pyrrolidone
e. 0.5 - 5% by weight of colloidal silicon dioxide
f. 0.1-5% by weight of magnesium stearate.

9. A process for preparing a pharmaceutical composition according to any one of the preceding claims, comprising the following steps;
a. granulating the mixture consisting of all alogliptin, mannitol, hydroxypropylcellulose and a part of microcrystalline cellulose and croscarmellose sodium with water,
b. drying and grinding the granule,
c. adding the remainder of microcrystalline cellulose and the remainder of croscarmellose sodium and colloidal silicon dioxide to the outer phase and mixing,
d. adding magnesium stearate to the mixture and then obtaining the final mixture,
e. performing the capsule filling process.
